# EUROPEAN PATENT SPECIFICATION

(11) **EP 4 595 815 B1**
(45) Date of publication and mention of the grant of the patent: **14.10.2026**
(21) Application number: 25151079.8
(22) Date of filing: 10.01.2025
(51) Int. Cl.: A46B 15/00, A61C 17/22

(54) **ORAL CARE DEVICE**
MUNDPFLEGEVORRICHTUNG
DISPOSITIF DE SOINS BUCCAUX

(30) Priority: 04.02.2024 CN 202420273824 U
(43) Date of publication of application: 06.08.2025
(73) Proprietor: Guangzhou Stars Pulse Co., Ltd., Guangzhou, Guangdong 510620 (CN)
(72) Inventor: ZHANG, Zhicheng, 510620 Guangzhou (CN)
(74) Representative: Metida

(56) References cited:
- WO-A1-2021/262528
- CN-U- 220 385 218
- US-A1- 2021 059 395
- US-A1- 2023 099 863
- US-A1- 2023 181 300

## Description

### TECHNICAL FIELD

The present disclosure relates to a technical field of oral care devices, and in particular to an oral care device.

### BACKGROUND

In the related art, a camera is disposed on a brush head of an electric toothbrush. Photos taken by the camera are displayed to realize a function of brushing while observing the photo, or the photos are displayed to indicate positions of teeth to be brushed, so that a user may better understand his/her brushing situation and thus better perform oral care. However, disposing the camera on the brush head makes a structure of the brush head complicated and leads to high replacement cost since the brush head is a consumable element. Moreover, the camera and the brush head are inserted into an oral cavity of the user, and the camera is easily blocked by saliva or foam, resulting in the camera being unable to obtain clear images.

Document D1 (US 2023/099863 A1) discloses a tracked toothbrush system. D1 discloses a toothbrush comprising at least one imager mounted on a handle. The imager is configured to capture, directly or via a mirror reflection, images of a user's facial features external to the oral cavity. D1 discloses that this system attempts to correlate the acquired images with a stored anatomical model to infer the position of a brush head within the mouth.

### SUMMARY

Embodiments of the present disclosure provide an oral care device that solves technical problems in the related art.

The embodiments of the present disclosure provide the oral care device. The oral care device includes a handle assembly, a care element, and an energy receiving device. The handle assembly includes a handle and an internal assembly. The handle defines a mounting cavity and a receiving port. The receiving port is communicated with the mounting cavity. The internal assembly includes an output shaft. The internal assembly is mounted in the mounting cavity and the output shaft extends out of the mounting cavity. The care element is connected to one end of the output shaft extending out of the mounting cavity. The energy receiving device is connected to the internal assembly or the handle, the energy receiving device is disposed corresponding to the receiving port, the energy receiving device is configured to acquire human body feature information, the energy receiving device has a predetermined receiving range, and the care element is disposed outside the predetermined receiving range.

In some embodiments, the oral care device further includes an energy transmitting device. The energy transmitting device is connected to the internal assembly or the handle. The energy transmitting device is disposed corresponding to the receiving port. The energy transmitting device is configured to transmit energy to a human body. The energy receiving device at least receives reflected energy of the energy transmitted by the energy transmitting device. The energy transmitting device has a predetermined transmitting range. The care element is disposed outside the predetermined transmitting range.

In some embodiments, the predetermined transmitting range is within the predetermined receiving range.

In some embodiments, the energy receiving device includes a camera, a photoelectric sensor, a sonic sensor or an infrared sensor.

In some embodiments, the handle includes a transition section and a holding section. The transition section includes a first connecting end and a second connecting end, and the second connecting end of the transition section is closer to the care element than the first connecting end of the transition section. The holding section is connected to the first connecting end of the transition section. Areas of cross sections, perpendicular to a length direction of the oral care device, of the holding section gradually decrease from the first connecting end of the transition section to the second connecting end of the transition section. The receiving port is disposed in the transition section, the receiving port is disposed in the holding section, or the receiving port is disposed at a junction of the transition section and the holding section. The receiving port is spaced apart from the care element.

In some embodiments, the receiving port is disposed in the transition section, and the receiving port is flush with an outer surface of the transition section.

In some embodiments, the receiving port is disposed in the holding section or the receiving port is disposed at a junction of the transition section and the holding section. The receiving port protrudes from an outer surface of the handle.

In some embodiments, the handle includes a top end close to the care element and a bottom end away from the care element. A distance between the receiving port and the top end of the handle is not greater than 40 mm, or a distance between the receiving port and the bottom end of the handle is not greater than 40 mm.

In some embodiments, the handle assembly further includes a protruding structure. The protruding structure protrudes from an outer peripheral wall of the handle along a radial direction of the handle. The receiving port is defined in a surface of the protruding structure facing the care element.

In some embodiments, the oral care device includes a light-permeable piece. The light-permeable piece covers on the receiving port to seal the receiving port. The light-permeable piece is inclined to guide liquid or foam to flow out of the light-permeable piece.

In some embodiments, the oral care device includes a light-permeable piece. The light-permeable piece covers on the receiving port to seal the receiving port. A hydrophobic film is coated on an outer surface of the light-permeable piece.

In some embodiments, the energy receiving device includes a camera. The camera directly faces the receiving port, and an inclined angle of the camera is same as an inclined angle of the light-permeable piece.

In some embodiments, the energy receiving device includes a camera. The camera defines an optical axis, the output shaft defines an axis, an included angle between the optical axis and the axis is not less than one-half of a field angle of the camera and not greater than a sum of one-half of the field angle of the camera and 10.

In some embodiments, the energy receiving device includes a camera. The camera defines an optical axis. The optical axis is inclined outward relative to the care element.

In some embodiments, the oral care device further includes a multi-axis sensor, and the multi-axis sensor is configured to obtain attitude information of the oral care device.

In some embodiments, in a circumferential dimension of the handle, a surface where a button is disposed or a care-oriented surface of the care element is defined as a front side of the handle, and the energy receiving device is disposed on a rear side of the handle.

In some embodiments, the energy receiving device includes a camera. The oral care device further includes a light source. The light source is disposed on the handle and is configured to supplement light for the camera, and/or the light source is turned on and off synchronously with the camera.

In some embodiments, the energy receiving device includes a camera. The internal assembly includes a bracket. The camera is disposed on the bracket. A light incident surface of the camera directly faces the receiving port.

In some embodiments, the oral care device further includes a controller. The controller is disposed in the handle and is electrically connected to the energy receiving device. The controller is configured to determine a position of the care element in an oral cavity based on the human body feature information acquired by the energy receiving device. Alternatively, the oral care device further includes a communication module. The communication module is configured to send the human body feature information acquired by the energy receiving device to an external terminal. The external terminal is configured to determine the position of the care element in the oral cavity based on the human body feature information acquired by the energy receiving device.

In some embodiments, the handle further includes a mounting opening communicated with the mounting cavity. The energy receiving device is disposed on the internal assembly. The energy receiving device and the internal assembly are mounted in the mounting cavity through the mounting opening.

Since the energy receiving device is connected to the internal assembly or the handle, the energy receiving device is separately disposed from the care element. When the care element is replaced, the energy receiving device does not need to be replaced synchronously, thereby reducing replacement cost of the care element. Moreover, when brushing teeth, the energy receiving device is not inserted into the oral cavity of a user, so the energy receiving device is not blocked by mouthwash or foam. In addition, by configuring the predetermined receiving range of the energy receiving device outside the care element, an inclined angle of the energy receiving device is effectively reduced, which reduces a size of the energy receiving device protruding from the outer surface of the handle. Further, under a same size, a small inclined angle of the energy receiving device reduces a probability of the energy receiving device being blocked by the handle when acquiring human body feature information.

### BRIEF DESCRIPTION OF DRAWINGS

In order to clearly describe technical solutions in the embodiments of the present disclosure, the following will briefly introduce the drawings that need to be used in the description of the embodiments or the prior art. Apparently, the drawings in the following description are merely some of the embodiments of the present disclosure, and those skilled in the art are able to obtain other drawings according to the drawings without contributing any inventive labor.
FIG. 1 is a structural schematic diagram of an oral care device according to one embodiment of the present disclosure.
FIG. 2 is a rear side schematic diagram of the oral care device according to one embodiment of the present disclosure.
FIG. 3 is a cross-sectional schematic diagram of the oral care device taken along a line A-A shown in FIG. 2.
FIG. 4 is an enlarged schematic diagram of area B shown in FIG. 3.
FIG. 5 is a schematic diagram of a shooting range of the oral care device according to one embodiment of the present disclosure.
FIG. 6 is a schematic diagram of a range of a field angle of an energy receiving device according to another embodiment of the present disclosure.
FIG. 7 is a schematic diagram of the range of the field angle of the energy receiving device according to another embodiment of the present disclosure.
FIG. 8 is a schematic diagram of the range of the field angle of the energy receiving device according to another embodiment of the present disclosure.
FIG. 9 is a schematic diagram of the range of the field angle of the energy receiving device according to another embodiment of the present disclosure.
FIG. 10 is a schematic diagram of the range of the field angle of the energy receiving device according to another embodiment of the present disclosure.
FIG. 11 is a schematic diagram of the shooting range of the oral care device according to another embodiment of the present disclosure.
FIG. 12 is a schematic diagram of the shooting range of the oral care device according to another embodiment of the present disclosure.
FIG. 13 is a schematic diagram of the shooting range of the oral care device according to another embodiment of the present disclosure.
FIG. 14 is a schematic diagram of the shooting range of the oral care device according to another embodiment of the present disclosure.
FIG. 15 is a schematic diagram of the shooting range of the oral care device according to another embodiment of the present disclosure.
FIG. 16 is a structural schematic diagram of the oral care device according to another embodiment of the present disclosure.
FIG. 17 is an enlarged schematic diagram of area C shown in FIG. 16.
FIG. 18 is a structural schematic diagram of a handle assembly according to one embodiment of the present disclosure.

### DETAILED DESCRIPTION

In order to make objectives, technical solutions, and advantages of the present disclosure clearer, the following further describes the present disclosure in detail with reference to accompanying drawings and embodiments. It should be understood that the specific embodiments described here are only used to explain the present disclosure, but not to limit the present disclosure.

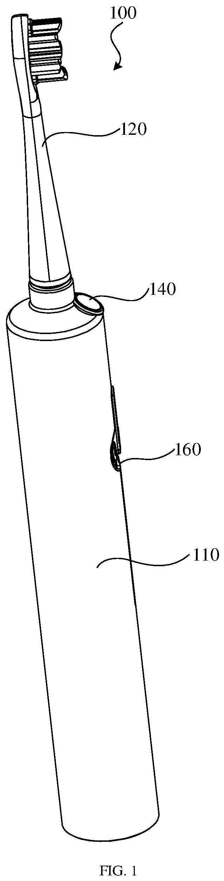

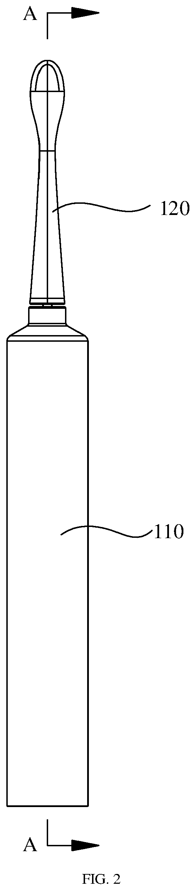

As shown in FIGS. 1-4, the embodiments of the present disclosure provide an oral care device 100. The oral care device 100 includes a handle assembly 110, a care element 120, and an energy receiving device 130.

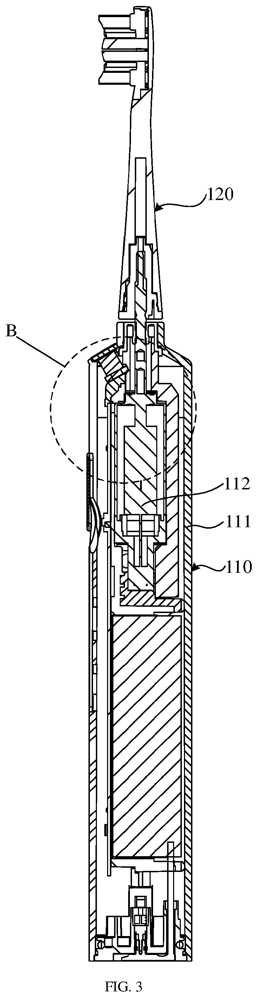

As shown in FIGS. 3-4, the handle assembly 110 includes a handle 111 and an internal assembly 112.

The handle 111 is configured to accommodate the internal assembly 112, and the handle 111 is convenient for a user to hold. The handle 111 defines a mounting cavity 111a and a receiving port 111b. The receiving port 111b is communicated with the mounting cavity 111a. The handle 111 may be made of plastic, so that the handle 111 is light and labor-saving for the user to hold. Moreover, the handle 111 made of plastic is allowed to be integrally formed, so the handle 111 has a good waterproof effect and is enabled to form into a complex shape.

The internal assembly 112 comprising an output shaft 1121. The internal assembly 112 is mounted in the mounting cavity 111a and the output shaft 1121 extends out of the mounting cavity 111a. That is, the output shaft 1121 of the internal assembly 112 passes through an interior of the handle 111 to an outside of the handle 111 to be connected to the care element 120.

A specific type of the care element 120 is related to a type of the oral care device 100. The oral care device 100 may be, for instance, an electric toothbrush, an oral irrigator, an oral detection device, etc. When the oral care device 100 is the electric toothbrush, the care element 120 is a brush head. When the oral care device 100 is the oral irrigator, the care element 120 is a nozzle. When the oral care device 100 is the oral detection device, the care element 120 is an endoscope head. Optionally, the care element 120 is the brush head. The care element 120 is connected to one end of the output shaft 1121 extending out of the mounting cavity 111a. The output shaft 1121 of the internal assembly 112 is configured to generate high-frequency reciprocating vibrations, thereby driving the brush head to generate high-frequency reciprocating vibrations. When the brush head contacts teeth of the user, food residues on the teeth of the user or tongue is removed by the high-frequency reciprocating vibrations of the brush head so as to achieve a purpose of cleaning an oral cavity of the user. It is understood that when the care element 120 is the nozzle, the output shaft 1121 is configured to convey high-pressure water flow. When the care element 120 is the endoscope head, the output shaft 1121 is configured to transmit light. Different types of care elements 120 are matched with different internal assemblys 112 to achieve different functions.

The energy receiving device 130 is connected to the internal assembly 112 or the handle 111, and is only necessary to fix the energy receiving device 130. For instance, the energy receiving device 130 is disposed on the handle 111, and in this case, the energy receiving device 130 is disposed separately from the internal assembly 112. Alternatively, the energy receiving device 130 is disposed on the internal assembly 112, and in this case, the energy receiving device 130 is served as a part of the internal assembly 112. The energy receiving device 130 is connected to the internal assembly 112 or the handle 111 by snapping, bonding, welding, etc. The energy receiving device 130 is disposed corresponding to the receiving port 111b. External energy enters the energy receiving device 130 through the receiving port 111b.

The receiving port 111b is formed on an outer surface of the handle 111. Optionally, the receiving port 111b is waterproofed to prevent water from entering the mounting cavity 111a of the handle 111 and affecting a normal operation of electronic devices such as the internal assembly 112 in the mounting cavity 111a. For instance, a shape of the receiving port 111b is matched with a shape of the energy receiving device 130, so that the energy receiving device 130 is able to block the receiving port 111b to seal the mounting cavity 111a. Alternatively, a connection position between the energy receiving device 130 and the receiving port 111b is sealed to seal the mounting cavity 111a.

The energy receiving device 130 receives energy through the receiving port 111b, and a type of the receiving port 111b is related to a type of the energy receiving device 130. The energy receiving device 130 is a camera, a photoelectric sensor, a sonic sensor or an infrared sensor. When the energy receiving device 130 is the camera, the receiving port 111b is configured to transmit visible light. The receiving port 111b may be open or the receiving port 111b is covered by a transparent cover. When the energy receiving device 130 is the photoelectric sensor, the receiving port 111b is configured to transmit the visible light or invisible light. The receiving port 111b is open or the receiving port 111b is covered by the transparent cover. When the energy receiving device 130 is the infrared sensor, the receiving port 111b is configured to transmit infrared light (transfer heat). The receiving port 111b is open or the receiving port 111b is covered by the transparent cover. When the energy receiving device 130 is the sonic sensor, the receiving port 111b is configured to transmit acoustic waves. The receiving port 111b needs to be open to prevent the acoustic waves from being blocked.

In some cases, the receiving port 111b is configured to mount the energy receiving device 130. After the energy receiving device 130 is assembled, the transparent cover is mounted on the receiving port 111b to cover the receiving port 111b, thereby facilitating an assembly of the energy receiving device 130 while ensuring sealing of the receiving port 111b.

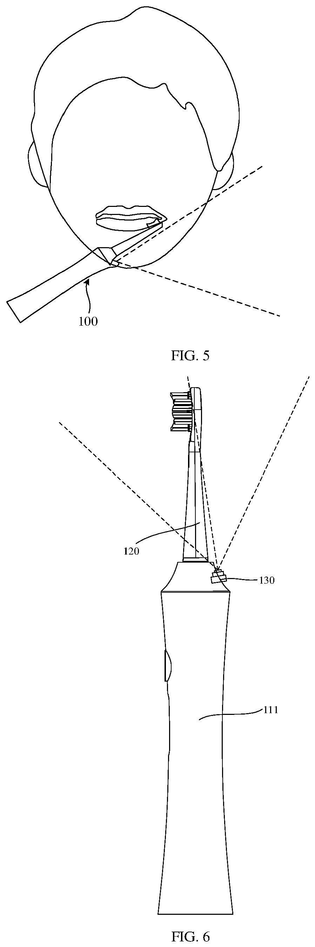

As shown in FIG. 5, the energy receiving device 130 is configured to receive energy reflected from a face of the user, and the energy may include the visible light, the invisible light (the(infrared light, etc.), the acoustic waves, ultrasonic waves, etc. The energy reflected from the face of the user is human body feature information acquired by the energy receiving device 130. For instance, by acquiring an optical image of the face of the user, image information of the face of the user is acquired. By acquiring a thermal image of the face of the user, thermal information of the face of the user is acquired. By acquiring an ultrasonic image of the face of the user, uneven facial features of the user are acquired. By analyzing above information, specific positions of the face, a mouth, a nose, ears, eyes, a neck, etc., of the user are identified, and then corresponding tooth position information is obtained through an artificial intelligence (AI) algorithm.

Optionally, the energy receiving device 130 is the camera, which is configured to capture images, and the images may be images of human features (including the face, the mouth, the nose, the ears, the eyes, the neck, etc.). According to the images captured by the camera, combined with the AI algorithm, a position of the care element 120 in the oral cavity of the user is relatively accurately determined. According to different determined positions, brushing conditions of brushing positions of the user are monitored and indicated. For instance, whether a brushing time of each of the brushing position is up to standard is determined, whether a brushing strength and a brushing amplitude of each of the brushing position are standardized is determined, etc., so as to prompt the user or output a correct guidance to the user. For example, when the user misses brushing part of tooth surfaces, or the brushing time is not enough, the oral care device 100 may prompt the user, thereby reducing a risk of tooth decay of the user. For another example, when the brushing time of part of the tooth surfaces is not enough, the user is prompted to continue brushing the part of tooth surfaces, or when the brushing time of a certain tooth surface reaches a predetermined time, the user is reminded to change a brushing position thereof.

The energy receiving device 130 has a predetermined receiving range. The predetermined receiving range is a maximum range in which the energy receiving device 130 is allowed to receive the energy. It is understood that the predetermined receiving range of the energy receiving device 130 is generally limited, so the energy receiving device 130 is difficult to receive information in every direction. When an orientation of the energy receiving device 130 changes, an energy information receiving area changes. For example, when the energy receiving device 130 is the camera, the predetermined receiving range is a field angle of the camera. Objects within the field angle are captured by the camera, and objects outside the field angle are unable to be captured by the camera.

The care element 120 is disposed outside the predetermined receiving range. That is, when the oral care device 100 is used, energy reflected by the care element 120 is unable to be received by the energy receiving device 130, and the energy receiving device 130 is only able to receive the energy reflected by the face of the user. The oral care device 100 identifies the teeth position only through the energy reflected by the face of the user. As shown in FIG. 6, the energy receiving device 130 is disposed toward the care element 120. That is, in order to ensure that the energy receiving device 130 is not blocked by the handle 111, the energy receiving device 130 needs to be inclined inward relative to the care element 120, and the energy receiving device 130 is partially exposed outside the handle 111. Therefore, the energy receiving device 130 protrudes higher from the handle 111.

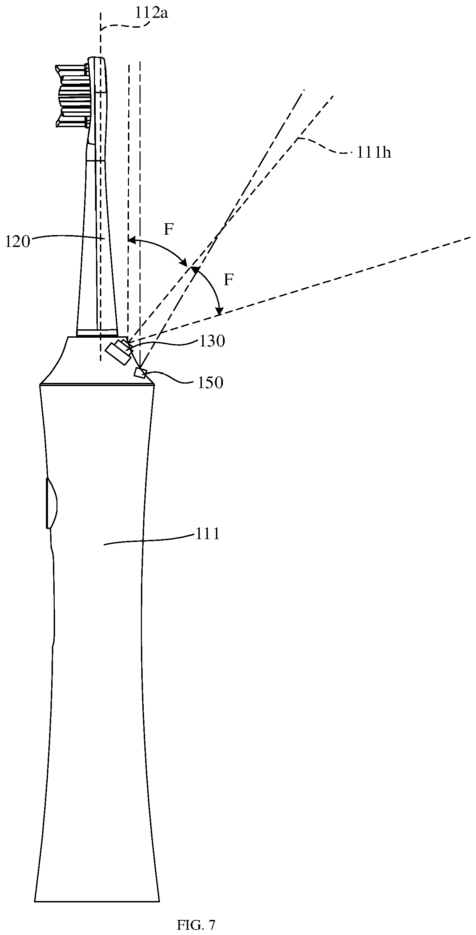

Alternatively, as shown in FIG. 7, the care element 120 is disposed outside the predetermined receiving range of the energy receiving device 130, and thus the energy receiving device 130 needs to be inclined outward relative to the care element 120. After the energy receiving device 130 is inclined outward, shielding of the energy receiving device 130 by the handle 111 is greatly reduced, and the energy receiving device 130 is enabled to be mounted at a more internal position of the handle 111. That is, the energy receiving device 130 is slightly protruded from the handle 111 or the energy receiving device 130 is not protruded relative to the handle 111.

Based on the oral care device 100 of the embodiments of the present disclosure, since the care element 120 is disposed outside the predetermined receiving range, the energy receiving device 130 does not need to be disposed toward the care element 120, and a position of the energy receiving device 130 on the handle assembly is more flexible determined. Moreover, since the care element 120 is disposed outside the predetermined receiving range, the care element 120 does not block the energy reflected by the face of the user, so that the energy receiving device 130 is allowed to receive more human body feature information. In addition, since the care element 120 is disposed outside the predetermined receiving range, the energy receiving device 130 generally only receive the human body feature information in a half of the predetermined receiving range close to the care element 120, thereby reducing an amount of calculation. When the energy receiving device 130 is disposed on a circumferential surface of the handle 111, the energy receiving device 130 is disposed at a large inclined angle, so that the energy receiving device 130 is slightly protruded from the handle 111 and a thickness of the handle 111 in a circumferential direction is small.

It should be noted that when the energy receiving device 130 is the camera, although a protruding height of the camera on the handle 111 may be reduced by increasing the field angle of the camera, the larger the field angle of the camera, the more serious the deformation of human features close to the camera that are display by the image taken by the camera, thereby increasing a difficulty of recognizing the image.

As shown in FIG. 7, in some embodiments, the oral care device 100 further includes an energy transmitting device 150. The energy transmitting device 150 is connected to the internal assembly 112 or the handle 111, only if the energy transmitting device is fixed. For example, the energy transmitting device 150 is disposed on the handle 111, and in this case, the energy transmitting device 150 is disposed separately from the internal assembly 112. Alternatively, the energy transmitting device 150 is disposed on the internal assembly 112, and in this case, the energy transmitting device 150 is served as a part of the internal assembly 112. The energy transmitting device 150 is connected to the internal assembly 112 or the handle 111 by snapping, bonding, welding, etc. The energy transmitting device 150 may be disposed separately from the energy receiving device 130 or may be disposed integrally with the energy receiving device 130.

The energy transmitting device 150 is configured to transmit the energy to the human body, and the energy is reflected after contacting the human body. The energy transmitting device 150 is disposed corresponding to the receiving port 111b.

The energy transmitted by the energy transmitting device 150 is reflected by the face of the user to form reflected energy, and the reflected energy passes through the receiving port 111b and is received by the energy receiving device 130. The care element 120 is disposed outside a predetermined transmitting range. That is, the care element 120 does not reflect energy, and thus the energy receiving device 130 does not receive the energy reflected by the care element120. By providing the energy transmitting device 150, detection reliability is improved and an influence of ambient light is reduced. A type of the energy emitting device 150 is reasonably set, so that the energy receiving device 130 does not obtain an image of a complete human body, thereby reducing a risk of privacy leakage. The energy transmitting device 150 and the energy receiving device 130 may be encapsulated in one component and presented in a form of one component to ensure assembly consistency and relative position stability of the energy transmitting device 150 and the energy receiving device 130.

Optionally, when the energy receiving device 130 is the sonic sensor, the energy transmitting device 150 is configured to transmit the acoustic waves. The acoustic waves are reflected from the face of the user and are received by the sonic sensor.

When the energy receiving device 130 is the photoelectric sensor, the energy transmitting device 150 is configured to transmit light waves. The light waves are reflected from the face of the user and are received by the photoelectric sensor. Alternatively, no energy transmitting device 150 is provided, and the photoelectric sensor receives the light waves reflected from the face of the user in nature.

When the energy receiving device 130 is the infrared sensor, the energy transmitting device 150 is configured to transmit the infrared light. The infrared light is reflected from the face of the user and is received by the infrared sensor. Alternatively, no energy transmitting device 150 is provided, and the infrared sensor receives infrared rays emitted by the face of the user. Alternatively, the infrared sensor receives reflected infrared rays from the face of the user in nature.

When the energy receiving device 130 is the camera, the energy transmitting device 150 is configured to transmit the visible light. The visible light is reflected from the face of the user and is received by the camera. Alternatively, no energy transmitting device 150 is provided, and the camera receives reflected natural light from the face of the user.

As shown in FIG. 7, in some embodiments, the predetermined transmitting range is within the predetermined receiving range. That is, the predetermined receiving range is not less than the predetermined transmitting range. The predetermined receiving range is a range formed by dash-dash lines on two sides of an optical axis 111h shown in FIG. 7. The predetermined transmitting range is a range formed by dot-dash lines in FIG. 7. Since the face of the user commonly appears in a portion of the predetermined receiving range close to the care element 120, the predetermined transmitting range only needs to cover the portion of the predetermined receiving range close to the care element 120. That is, the energy emitted by the energy transmitting device 150 is able to cover the face of the user, thereby reducing a specification requirement for the energy transmitting device 150 and making a location of the energy transmitting device 150 more flexible. In this way, the energy receiving device 130 is ensured to receive more reflected energy of the human body/face, more features are identified, and a corresponding oral care position is more accurately analyzed and acquired.

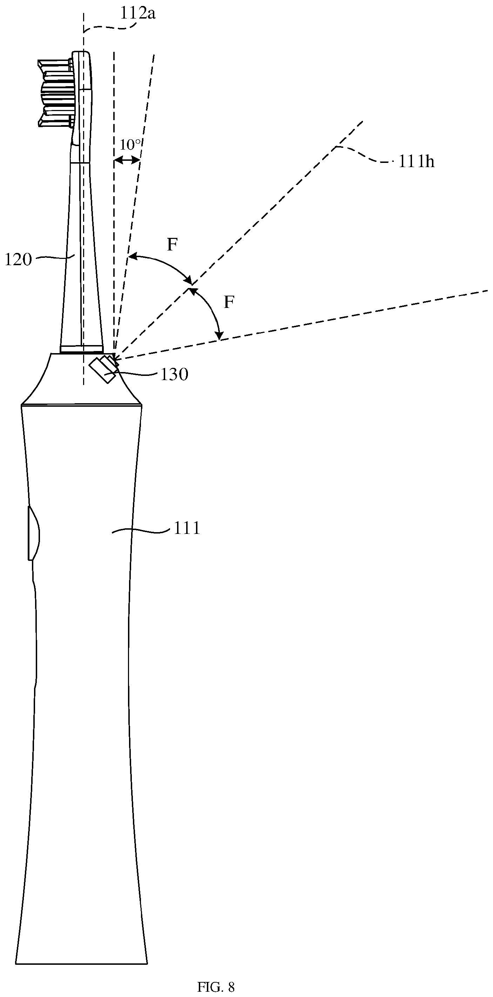

As shown in FIGS. 7 and 8, in some embodiments, the energy receiving device 130 is the camera. The camera defines an optical axis 111, the output shaft 1121 defines an axis 112a, an included angle between the optical axis 111 and the axis is not less than F degrees and is not greater than F+10 degrees. F is one-half of the field angle of the camera.

When the included angle between the optical axis 111h and the axis 112a is equal to F degrees, an edge line of the field angle of the camera is parallel to the axis 112a of the output shaft 1121. When the user puts the care element 120 into the oral cavity, the camera can capture enough face features of the user. If the included angle is too large, the camera captures less face features of the use, which affects the accuracy of the judgment.

In some embodiments of the present disclosure, the energy receiving device 130 includes the camera, the camera has the optical axis 111h, and the optical axis 111h is deviated from the care element 120, so that the care element 120 is located outside the predetermined receiving range. The optical axis 111h is deviated from the care element 120, that is, the camera is tilted relative to the output shaft 1121, so that a portion of the camera protruding from the handle 111 is small or the camera does not protrude from the handle 111, thereby reducing a size of the handle 111 in a redial direction. When the camera does not protrude from the handle 111, the camera is disposed inside the handle 111, that is, the camera is hidden, so the camera is protected and service life thereof increases.

In some embodiments of the present disclosure, the optical axis 111h is inclined outward relative to the care element 120, that is, the optical axis 111h is deflected in a direction away from the care element 120, so that the camera is inclined relative to the output axis 1121. Therefore, the camera is less protruded in the radial direction of the handle 111 and an arrangement of the camera is more flexible.

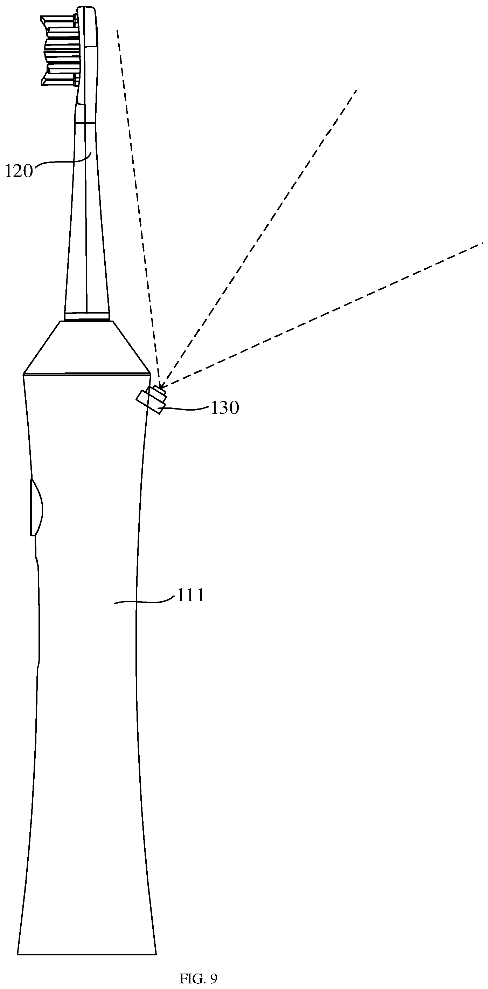

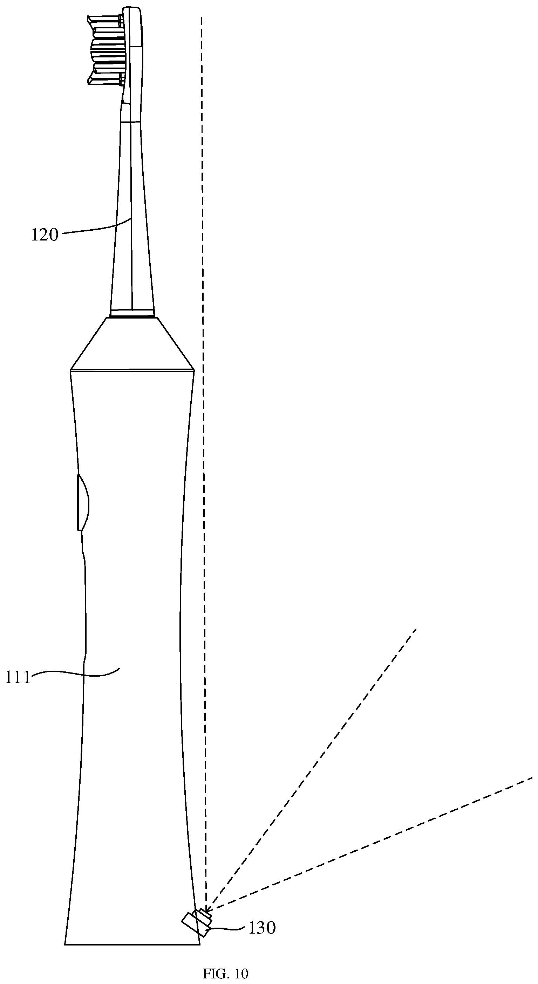

As shown in FIGS. 9 and 10, in some embodiments, the handle 111 includes a top end close to the care element 120 and a bottom end away from the care element 120. A distance between the receiving port 111b and the top end of the handle 111is not greater than 40 mm, or a distance between the receiving port 111b and the bottom end of the handle 111is not greater than 40 mm.

The top end and the bottom end of the handle 111 are areas that are not held by the user when using the oral care device 100. Therefore, the receiving port 111b is defined in the top end or the bottom end of the handle 111 and is not blocked by the hand. Further, discomfort of holding caused by a protrusion arrangement of a light inlet thereof in the circumferential direction is avoided.

Since the energy receiving device 130 is allowed to be disposed at a larger inclination angle, the energy receiving device 130 protrude less radially in the handle 111, a probability that the energy receiving device 130 is blocked by the handle 111 is smaller, and the arrangement of the energy receiving device 130 is more flexible. Compared with the energy receiving device 130 being disposed toward the care element120, under the same protrusion size, the energy receiving device 130 of the embodiment is able to be dispose at a position farther from the care element 120. For instance, the energy receiving device 130 may be disposed at the bottom end of the handle 111. The farther the energy receiving device 130 is from the care element 120, the lower the probability of the energy receiving device 130 being blocked by liquid or the foam.

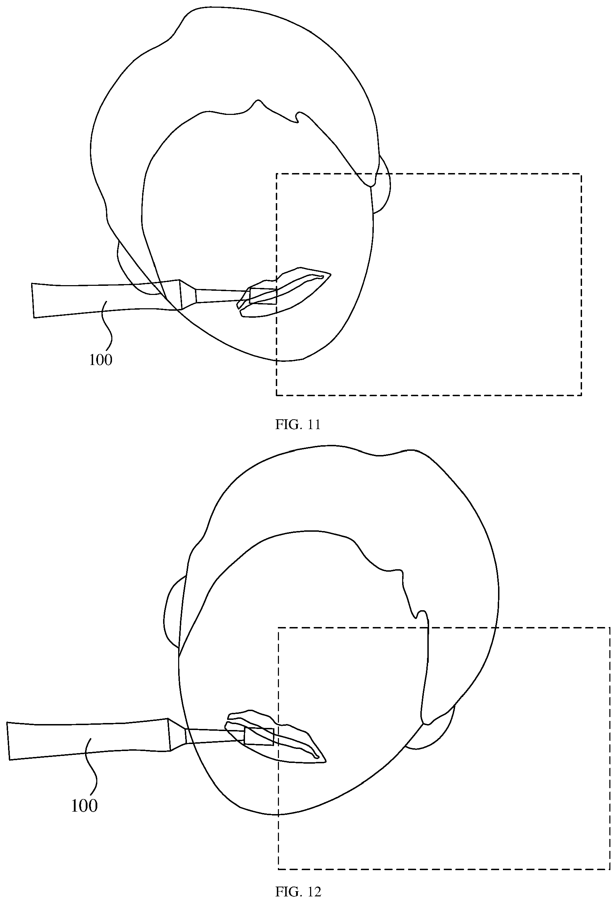

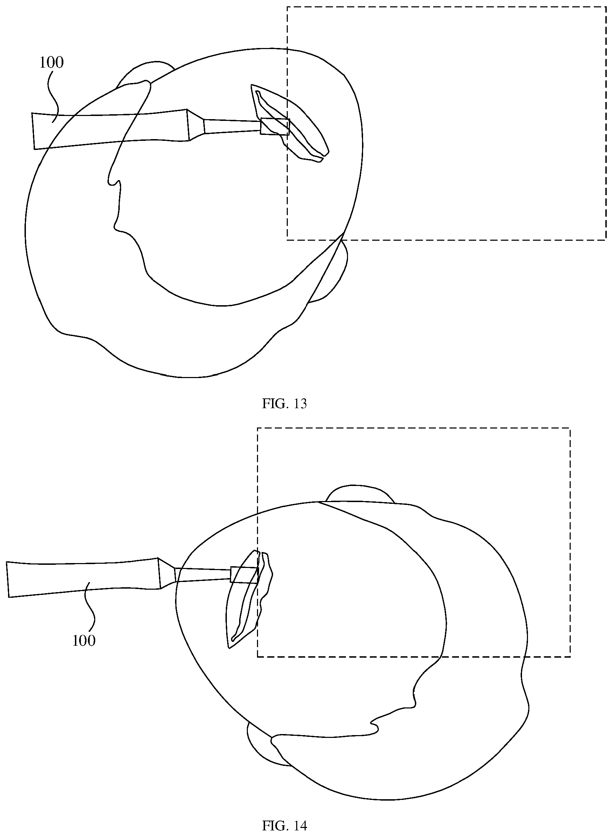

As shown in FIGS. 11-14, the dotted box represents an Identification range of the oral care device 100. FIG. 11 is a schematic diagram of using the oral care device 100 to clean upper teeth in a left side of the oral cavity, FIG. 12 is a schematic diagram of using the oral care device 100 to clean lower teeth in the left side of the oral cavity, FIG. 13 is a schematic diagram of using the oral care device 100 to clean upper molars in the left side of the oral cavity. FIG. 14 is a schematic diagram of using the oral care device 100 to clean lower molars in the left side of the oral cavity. According to FIGS. 11-14, it is noted that the oral care device 100 is able to capture various facial images, thereby ensuring that the oral care device 100 has a high recognition accuracy rate. Since the left side of the oral cavity and the right side of the oral cavity are symmetrical, the oral care device 100 also has a high recognition accuracy rate when cleaning symmetrical positions of the left side of the oral cavity.

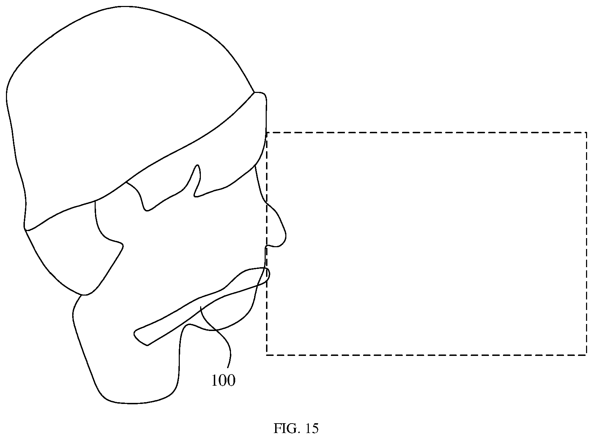

As shown in FIG. 15, FIG. 15 is a schematic diagram of using the oral care device 100 to clean front teeth. In this case, since the energy receiving device 130 is roughly parallel to the face, the oral care device 100 capture few facial images and the recognition accuracy rate is relatively low.

In some embodiments, the oral care device 100 further includes a multi-axis sensor, and the multi-axis sensor is configured to obtain attitude information of the oral care device 100. The multi-axis sensor may include, but is not limited to, a three-axis acceleration sensor and/or a three-axis gyroscope sensor. The attitude information may include, but is not limited to, an acceleration and a direction of the oral care device in three-dimensional space and/or a rotation angle, a rotation speed and a rotation axis of the oral care device in the three-dimensional space.

Different attitude information corresponds to different attitudes of the oral care device 100. Through multiple simulation tests, different attitude information are matched with different attitudes of the oral care device 100. Different attitude of the oral care device 100 represent different positions for cleaning the oral cavity. Through multiple simulation tests, different attitudes of the oral care device 100 are matched with different positions for cleaning the oral cavity, so that it is determined which part of the oral cavity is currently cleaned by the oral care device 100 based on the attitude information.

A more accurate position of the care element is obtained by providing the attitude information and human body feature information as additional input information to an AI processing model. Alternatively, when there is few human body feature information, a brushing position is determined in combination with the attitude information. Based on dual calibration of the human body feature information and the attitude information, a current oral care position corresponding to the care element 120 is more accurately identified.

For instance, the human body feature information is configured to determine whether the care element 120 is currently in an upper tooth area or a lower tooth area, and the attitude information is configured determine an orientation of the care element 120, thereby further accurately determining whether the care element 120 is currently in a left tooth area, a right tooth area, or a middle tooth area. Finally, the determined upper or lower tooth area is combined with determined left tooth area, right tooth area, or middle tooth area to obtain a more accurate current oral care position corresponding to the care element 120.

Alternatively, after obtaining the human body feature information and the attitude information, a first current oral care position corresponding to the care element 120 is determined based on the human body feature information, a second current oral care position corresponding to the care element 120 is determined based on the attitude information, and then the current oral care position corresponding to the care element 120 is determined based on the first current oral care position and the second current oral care position. For example but not limited to, the current oral care position to which the care element 120 finally corresponds is an overlapping position between the first current oral care position and the second current oral care position.

In some embodiments, the handle 111includes a transition section 1111 and a holding section 1112. The transition section 1111 includes a first connecting end 1113 and a second connecting end 1114, and the second connecting end 1114 of the transition section 1111 is closer to the care element 120 than the first connecting end 1113 of the transition section 1111. The holding section 1112 is connected to the first connecting end 1113 of the transition section 1111. Areas of cross sections, perpendicular to a length direction EE of the oral care device 100, of the holding section 1112 gradually decrease from the first connecting end 1113 of the transition section 1111 to the second connecting end 1114 of the transition section 1111. It should be noted that the areas of the cross sections of the transition section 1111 perpendicular to the length direction EE of the oral care device 100 may decrease smoothly or in a stepwise manner. The receiving port 111b is disposed in the transition section 1111. The receiving port 111b is spaced apart from the care element 120.

In the oral care device 100 of the embodiment of the present disclosure, the transition section 1111 gradually tapers from the first connecting end 1113 to the second connecting end 1114 of the transition section 1111, so that the transition section 1111 is prevented from blocking the energy entering the receiving port 111b as much as possible. Therefore, the energy receiving device 130 may better obtain the energy. Moreover, the water on the care element 120 is allowed to flow smoothly from the second connecting end 1114 to the first connecting end 1113 of the transition section 1111, and then being discharged along the holding section 1112, so that the water on the care element 120 is not easy to block the receiving port 111b. The receiving port 111b is disposed in the transition section 1111, or the receiving port 111b is disposed in the holding section 1112, or the receiving port 111b is disposed at a junction of the transition section 1111 and the holding section 1112, which is determined according to actual needs.

In some embodiments, the receiving port 111b is disposed in the transition section 1111, and the receiving port 111b is flush with an outer surface of the transition section 1111. That is, an outer surface of the transition section 1111 is a smooth surface, so that a boundary of the transition section 1111 and the receiving port 111b is not easy to hide dirt and grime, and the liquid and the foam are difficult to adhere to the transition section 1111.

In some embodiments, the receiving port 111b is disposed in the holding section 1112, or the receiving port 111b is disposed at a junction of the transition section 1111 and the holding section 1112. The receiving port 111b protrudes from an outer surface of the handle, so that the receiving port 111b is not blocked by the handle 111.

In some embodiments, the handle assembly 110 further includes a protruding structure 113. The protruding structure 113 protrudes from an outer peripheral wall of the handle 111along the radial direction of the handle 111. A protruding position of the protruding structure113 may be disposed on the top end, the bottom end, or the middle portion of the handle 111, which is not limited thereto. The protruding structure 113 and the handle 111 may be integrally formed, thereby reducing a manufacturing process and improving waterproof performance of the protruding structure113 and the handle 111 as a whole. Alternatively, the protruding structure 113 and the handle 111 are separately provided, so that the protruding structure 113 of different specifications is able to be flexibly matched with the handle 111 of different specifications, and a connection method between the protruding structure 113 and the handle 111 may be clamping, bonding, welding, etc. The receiving port 111b is defined in a surface of the protruding structure 113 facing the care element 120.

In the oral care device 100 of the embodiment of the present disclosure, by providing the protruding structure 113, the energy entering the receiving port 111b is not blocked by the handle 111, so that the energy receiving device 130 better receives the human body feature information.

In some embodiments of the present disclosure, the receiving port 111b is defined in one side of the protruding structure 113 away from the handle 111 in the radial direction of the handle 111. That is, when a size of the protruding structure 113 remains unchanged, the receiving port 111b is disposed as far away from the handle 111 as possible, and a distance between the receiving port 111b and the handle 111 is relatively large, so that the liquid on the handle 111 is difficult to flow to the receiving port 111b, and the handle 111 does not excessively block the receiving port 111b.

In the oral care device 100 of the embodiment of the present disclosure, the receiving port 111b is away from the handle 111, which make the liquid on the handle 111 being difficult to flow to the receiving port 111b, thereby reducing a probability of the liquid flowing to the receiving port 111b and improving waterproof performance of the receiving port 111b. Moreover, the receiving port 111b is away from the handle 111, which makes a distance between the energy receiving device 130 and the handle 111 as large as possible, thereby facilitating the energy receiving device 130 to receive the human body feature information.

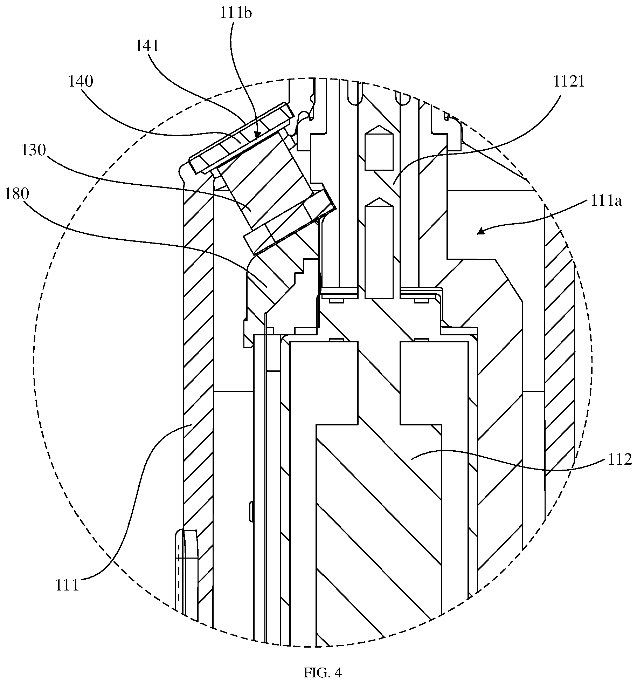

As shown in FIG. 4, in some embodiments, the energy receiving device 130 includes a camera, and the oral care device 100 includes a light-permeable piece 140. The light-permeable piece 140 covers on the receiving port 111b to seal the receiving port 111b. The light-permeable piece 140 may be disposed on the outer surface of the handle 111. The light-permeable piece 140 and the handle 111 may be integrally formed, thereby simplifying the manufacturing process, and the light-permeable piece 140 and the handle 111 as a whole have a good waterproof effect. Alternatively, the light-permeable piece 140 and the handle 111 may be separately disposed. For example, the light-permeable piece 140 and the handle 111 are connected by clamping, bonding, welding, etc., so as to realize flexible assembly of the light-permeable piece 140 of different specifications and the handle 111 of different specifications.

The light-permeable piece 140 may be a light-transmitting plastic sheet or a light-transmitting glass sheet. It should be noted that the light-permeable piece 140 may be a flat glass sheet or a curved glass sheet having a bending force on light, so as to cooperate with the energy receiving device 130 to achieve a specific optical effect, such as wide-angle shooting.

An outer surface of the light-permeable piece 140 is coated with a hydrophobic film 141, and the hydrophobic film 141 may be a light-transmitting nano film. It is understood that the outer surface of the light-permeable piece 140 may be coated with other types of coatings, such as an optical coating.

In the oral care device 100 of the embodiment of the present disclosure, the outer surface of the light-permeable piece 140 is coated with the hydrophobic film 141, so that the outer surface of the light-permeable piece 140 has a large water contact angle, and the liquid such as water droplets are difficult to adhere to the outer surface of the light-permeable piece 140. Therefore, the outer surface of the light-permeable piece 140 is kept as clean as possible, reducing a negative impact of the liquid such as the water droplets on the shooting of the energy receiving device 130 while making the light-permeable piece 140 easy to clean. In addition, the liquid such as the water droplets are not easy to adhere to the outer surface of the light-permeable piece 140, which reduces the time the liquid stays on the light-permeable piece 140. When the light-permeable piece 140 and the handle 111 are integrally disposed, the hydrophobic film 141 further reduces a probability of water leakage at a junction of the light-permeable piece 140 and the handle 111.

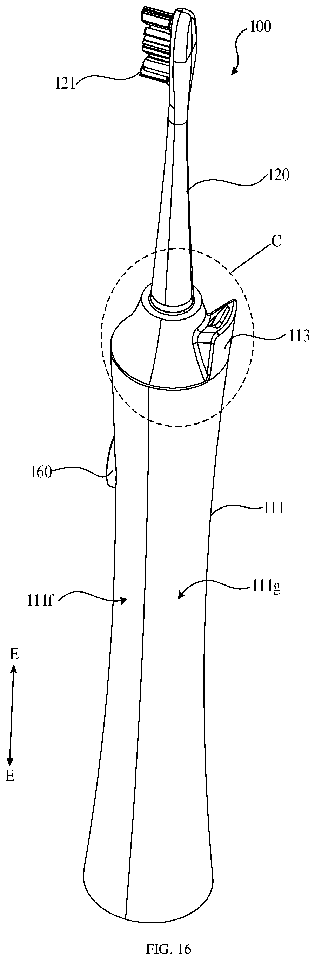

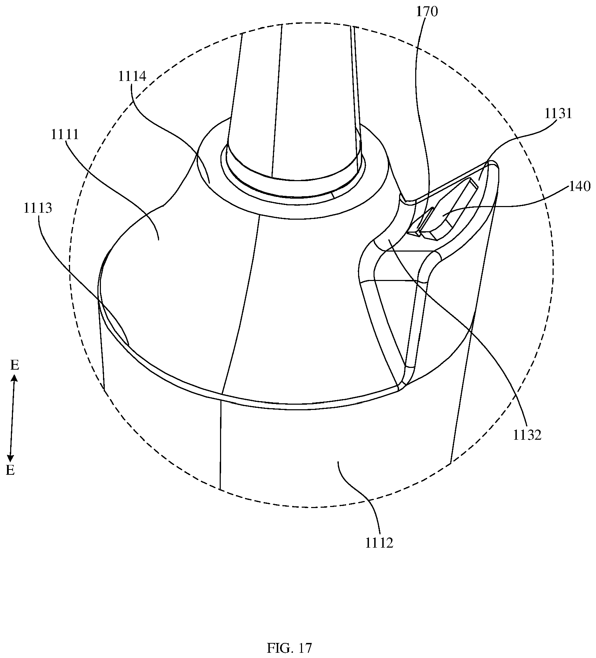

As shown in FIGS. 16 and 17, in some embodiments of the present disclosure, the light-permeable piece 140 is inclined to guide the liquid to flow out of the light-permeable piece 140. Optionally, in a common holding posture and a common placement state of the oral care device 100, the light-permeable piece 140 has an included angle with a horizontal plane, so as to facilitate the liquid such as the water droplets to slide off the outer surface of the light-permeable piece 140.

In the oral care device 100 of the embodiment of the present disclosure, the light-permeable piece 140 is inclined to guide the liquid to flow out of the light-permeable piece 140, so that the outer surface of the light-permeable piece 140 is kept as clean as possible, which reduces the negative impact of liquids such as the water droplets on the shooting of the energy receiving device 130. Moreover, a drainage effect produced by an inclined arrangement of the light-permeable piece 140 is relatively stable and reliable, and the drainage effect does not degrade after long-term use.

In some embodiments of the present disclosure, the camera directly faces the receiving port 111b, so that the camera is able to receive as much human body feature information as possible from the receiving port 111b. A size of the receiving port 111b is matched with a light incident surface of the camera. If the receiving port 111b is too large, too much stray light may enter the camera. If the receiving port 111b is too small, few facial image are captured by the camera. An inclined angle of the camera is same as an inclined angle of the light-permeable piece 140, so that the camera is tightly attached to the light-permeable piece 140, thereby reducing a volume of the camera and the handle 111 after assembly.

As shown in FIG. 16, in some embodiments, in a circumferential dimension of the handle 111, a surface where a button 160 is disposed or a care-oriented surface of the care element 120 is defined as a front side of the handle 111, and the energy receiving device 130 is disposed on a rear side of the handle.

For instance, the care element 120 includes bristles 121, a surface of the handle 111 facing the same direction as a brushing surface of the bristles 121 is defined as the front side 111g of the handle 111, and the receiving port 111b is disposed on the rear side 111f of the handle 111. In other words, the oral care device 100 includes the button 160, the button 160 is disposed on the front side 111g of the handle 111, and the receiving port 111b is disposed on the rear side 111f of the handle 111. It should be noted that in the embodiment, since a shape of the handle 111 is roughly cylindrical, the front side 111g and the rear side 111f of the handle 111 are relative concepts, the front side 111g and the rear side111f of the handle 111 are curved surfaces, and the front side 111g and the rear side111f of the handle 111 are connected. It should be noted that the button 160 and the receiving port 111b are not limited to being located opposite to each other on two sides in the length direction EE of the oral care device 100. For example, the button 160 is disposed at a middle portion or an upper middle portion of the front side 111g of the handle 111, and the receiving port 111b is disposed at atop position of the rear side111f of the handle 111.

In the oral care device 100 of the embodiment of the present disclosure, the energy receiving device 130 is disposed on the rear side of the handle 111. When brushing teeth, the energy receiving device 130 is on an outer side of the handle away from the human body. In other words, the energy receiving device 130 is farther away from the chin of the user, thereby reducing a probability of the energy receiving device 130 being blocked by the chin. In addition, the energy receiving device 130 is disposed on the rear side 111f of the handle 111. When the oral care device 100 is not in use, the care element 120 blocks an optical path of the energy receiving device 130, and it is difficult for the energy receiving device 130 to capture privacy images of the user. In addition, since the energy receiving device 130 is disposed toward the care element 120, when the oral care device 100 is placed on a sink, the energy receiving device 130 faces obliquely upward. That is, the energy receiving device 130 faces a ceiling of a bathroom, so the energy receiving device 130 is also difficult to capture the privacy images of the user.

As shown in FIG. 17, in some embodiments, the oral care device 100 further includes a light source 170. The light source 170 is disposed on the handle 111 or the protruding structure 113 (at this time, the light source 170 is equivalent to being indirectly disposed on the handle 111 through the protruding structure 113). The light source 170 is configure to fill in the light for the energy receiving device 130, so as to improve the clarity of the image when the energy receiving device 130 is shot in a dim environment. The light source 170 may be, for example, an LED lamp bead, which has relatively high brightness and is energy-saving. The light emitted by the light source 170 is optionally soft light, so as to reduce the stimulation to the eyes. The light source 170 is turned on or turned off synchronously with the energy receiving device 130 to prompt the user of a working status of the energy receiving device 130. Of course, the light source 170 may have other start-up conditions. For example, the light source 170 is configured to be turned on only when a brightness value of ambient light is lower than a predetermined threshold.

In the oral care device 100 of the embodiment of the present disclosure, the light source 170 supplements light for the energy receiving device 130, thereby increasing an amount of the light entering the energy receiving device 130, improving the shooting effect of the energy receiving device 130, and further expanding the use scenarios of the oral care device 100. Moreover, the light source 170 is turned on or turned off synchronously with the energy receiving device 130, that is, when the light source 170 is turned off, the energy receiving device 130 is turned off, and when the light source 170 is working, the energy receiving device 130 is working. Therefore, the user is able to determine whether the energy receiving device 130 is working according to the state of the light source 170, so that the user can know the working status of the energy receiving device 130 more intuitively, avoiding privacy disclosure when the user is unaware.

It is understood that when there is sufficient light, the light source 170 does not need to supplement the light all the time. In this case, the light source 170 only lights up for a certain period of time or flash a few times when the energy receiving device 130 is turned on, prompting the user that the energy receiving device 130 is turned on. Then, the energy receiving device 130 is kept turning on, but the light source 170 is turned off.

As shown in FIG. 4, in some embodiments, the internal assembly 112 includes: a bracket 180, a battery, a motor, a circuit board. The battery, the motor, and the circuit board are disposed on the bracket 180, and the energy receiving device 130 is disposed on the bracket 180. The light incident surface of the energy receiving device 130 directly faces the receiving port 111b. It should be noted that during assembly, the energy receiving device 130 is first assembled on the bracket 180, and then a whole component composed of the energy receiving device 130, the battery, the motor, the circuit board, and the bracket 180 is mounted in the mounting cavity 111a through the mounting opening 111e. The bracket 180 and the mounting cavity 111a include guiding structures. When the bracket 180 is assembled in place, the light incident surface of the energy receiving device 130 directly faces the receiving port 111b.

In the oral care device 100 of the embodiment of the present disclosure, the orientation of the energy receiving device 130 is limited by the bracket 180, so that the energy receiving device 130 directly faces the receiving port 111b, thereby ensuring that the field angle of the energy receiving device 130 is not blocked. Moreover, the light incident surface of the energy receiving device 130 is disposed as close to the receiving port 111b as possible, thereby making a structure of the oral care device 100 more compact.

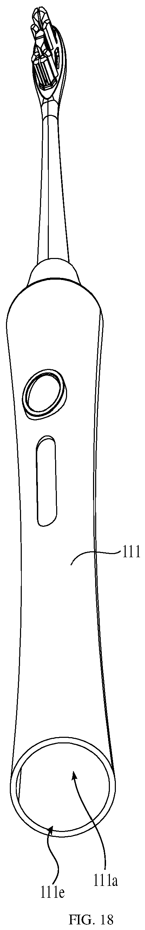

As shown in FIG. 18, in some embodiments, the handle 111further includes the mounting opening 111e communicated with the mounting cavity 111a. The energy receiving device 130 is disposed on the internal assembly 112. The energy receiving device 130 and the internal assembly are mounted in the mounting cavity 111a through the mounting opening 111e. The mounting opening 111e may be disposed at the bottom end of the handle 111, so that it is difficult for the liquid to flow back from the mounting opening to the mounting cavity 111a.

In the oral care device 100 of the embodiment of the present disclosure, the energy receiving device 130 is connected to the internal assembly 112, and the energy receiving device 130 and the internal assembly 112 are assembled together in the handle 111, thereby reducing assembly steps and improving assembly efficiency.

In some embodiments, the oral care device 100 further includes a controller disposed in the handle 111. For instance, the controller is disposed in the mounting cavity 111a of the handle 111. The controller is electrically connected to the energy receiving device 130. The controller is configured to determine a position of the care element 120 in the oral cavity based on the images (i.e., the human body feature images and the images of the care element 120) acquired by the energy receiving device 130., so as to calculate the current oral care position. Of course, the controller is further configured to control the opening and closing of the energy receiving device 130. The controller may be disposed on the circuit board.

Alternatively, the oral care device 100 further includes a communication module. The communication module is configured to send the images (i.e., the human body feature images and the images of the care element 120) acquired by the energy receiving device 130 to an external terminal. The external terminal is configured to determine the position of the care element 120 in the oral cavity based on the images acquired by the energy receiving device 130. The external terminal is further configured to determine a possible oral disease risk based on brushing records of the user. The communication module may be disposed on the circuit board.

In the oral care device 100 of the embodiment of the present disclosure, the controller or the external terminal is able to determine the position of the care element120 in the oral cavity based on the image acquired by the energy receiving device 130, and prompt the user. For example, the user is prompted through a display screen on the oral care device 100, so as to realize a function of brushing while watching, thereby helping the user to improve the brushing efficiency.

In the drawings of the embodiments, the same or similar numbers correspond to the same or similar components. In the description of the present disclosure, it should be understood that terms such as "upper", "lower", "left", "right", etc., indicate direction or position relationships shown based on the drawings, and are only intended to facilitate the description of the present disclosure and the simplification of the description rather than to indicate or imply that the indicated device or element must have a specific direction or constructed and operated in a specific direction. Therefore, the terms used to describe positional relationships in the drawings are only for illustrative purposes and cannot be construed as limitations of the present disclosure. For those of ordinary skill in the art, the specific meanings of the above terms can be understood according to specific circumstances.

The above are only some embodiments of the present disclosure and are not intended to limit the present disclosure.

## Claims

1. An oral care device (100), comprising:
a handle assembly (110);
a care element (120); and
an energy receiving device (130);
wherein the handle assembly (110) comprises a handle (111) and an internal assembly (112), the handle (111) defines a mounting cavity (111a) and a receiving port (111b), the receiving port (111b) is connected with the mounting cavity (111a), the internal assembly (112) comprises an output shaft (1121), the internal assembly (112) is mounted in the mounting cavity (111a) and the output shaft (1121) extends out of the mounting cavity (111a);
wherein the care element (120) is connected to one end of the output shaft (1121) extending out of the mounting cavity (111a);
wherein the energy receiving device (130) is connected to the internal assembly (112) or the handle (111), the energy receiving device (130) is disposed corresponding to the receiving port (111b), the energy receiving device (130) is configured to acquire human body feature information, the energy receiving device (130) has a predetermined receiving range, wherein the energy receiving device (130) comprises a camera, the camera defining an optical axis (111h), the output shaft (1121) defines an axis, wherein
the optical axis (111h) of the camera is deflected outward at an angle relative to the axis (112a) of the output shaft (1121) so as to be deviated from the care element (120) such that the care element (120) is disposed outside the predetermined receiving range.

2. The oral care device (100) according to claim 1, wherein the oral care device (100) further comprises an energy transmitting device (150), the energy transmitting device (150) is connected to the internal assembly (112) or the handle (111), and the energy transmitting device (150) is disposed corresponding to the receiving port (111b);
wherein the energy transmitting device (150) is configured to transmit energy to a human body, the energy receiving device (130) is configured to at least receive reflected energy of the energy transmitted by the energy transmitting device (150), the energy transmitting device (150) has a predetermined transmitting range, and the care element (120) is disposed outside the predetermined transmitting range.

3. The oral care device (100) according to claim 2, wherein the predetermined transmitting range is within the predetermined receiving range.

4. The oral care device (100) according to claim 1, wherein an included angle between the optical axis of the camera and the axis of the output shaft is not less than F degrees and not greater than F +10 degrees, and F is one-half of a field angle of the camera.

5. The oral care device (100) according to claim 1, wherein the optical axis of the camera is inclined outward relative to the care element (120), so that the care element (120) is disposed outside the predetermined receiving range.

6. The oral care device (100) according to claim 1, wherein the oral care device (100) further comprises a multi-axis sensor, the multi-axis sensor being configured to obtain attitude information of the oral care device.

7. The oral care device (100) according to claim 1, wherein in a circumferential dimension of the handle, a surface where a button is disposed or a care-oriented surface of the care element (120) is defined as a front side of the handle (111), and the energy receiving device (130) is disposed on a rear side of the handle (111).

8. The oral care device (100) according to claim 1, wherein the internal assembly (112) comprises: a bracket (180), the camera is disposed on the bracket (180), and a light incident surface of the camera directly faces the receiving port (111b).

9. The oral care device (100) according to claim 1, wherein the handle (111) further comprises a mounting opening (111e) connected with the mounting cavity (111a), the energy receiving device (130) is disposed on the internal assembly (112), and the energy receiving device (130) and the internal assembly (112) are mounted in the mounting cavity (111a) from the mounting opening (111e).

10. The oral care device (100) according to claim 1, wherein the handle (111) comprises a transition section (1111) and a holding section (1112), the transition section (1111) comprises a first connecting end (1113) and a second connecting end (1114), the second connecting end (1114) of the transition section (1111) is closer to the care element (120) than the first connecting end (1113) of the transition section (1111), the holding section (1112) is connected to the first connecting end (1113) of the transition section (1111), and areas of cross sections, perpendicular to a length direction of the oral care device (100), of the holding section (1112) gradually decrease from the first connecting end (1113) of the transition section (1111) to the second connecting end (1114) of the transition section (1111);
wherein the receiving port (111b) is disposed in the transition section (1111), the receiving port (111b) is disposed in the holding section (1112), or the receiving port (111b) is disposed at a junction of the transition section (1111) and the holding section (1112);
wherein the receiving port (111b) is spaced apart from the care element (120).

11. The oral care device (100) according to claim 1, wherein the handle assembly (110) further comprises a protruding structure (113), the protruding structure (113) protrudes from an outer peripheral wall of the handle (111) along a radial direction of the handle (111), and the receiving port (111b) is defined in a surface of the protruding structure (113) facing the care element (130).

12. The oral care device (100) according to claim 1, wherein the oral care device (100) comprises a light-permeable piece (140), the light-permeable piece (140) covers on the receiving port (111b) to seal the receiving port (111b), and the light-permeable piece (140) is inclined to guide liquid or foam to flow out of the light-permeable piece (140).

13. The oral care device (100) according to claim 1, wherein the oral care device (100) comprises a light-permeable piece (140), the light-permeable piece (140) covers on the receiving port (111b) to seal the receiving port (111b), and a hydrophobic film (141) is coated on an outer surface of the light-permeable piece (140).

14. The oral care device (100) according to claim 12 or claim 13, wherein the camera directly faces the receiving port (111b), and an inclined angle of the camera is same as an inclined angle of the light-permeable piece (140).

15. The oral care device (100) according to claim 1, wherein the oral care device (140) further comprises a controller, the controller is disposed in the handle (111) and is electrically connected to the energy receiving device (130), and the controller is configured to determine a position of the care element (120) in an oral cavity based on the human body feature information acquired by the energy receiving device (130); or
wherein the oral care device (100) further comprises a communication module, the communication module is configured to send the human body feature information acquired by the energy receiving device (130) to an external terminal, and the external terminal is configured to determine the position of the care element (120) in the oral cavity based on the human body feature information acquired by the energy receiving device (130).

## Patentansprüche

1. Mundpflegevorrichtung (100), umfassend:
eine Griffbaugruppe (110);
ein Pflegeelement (120); und
eine Energieempfangsvorrichtung (130);
wobei die Griffbaugruppe (110) einen Griff (111) und eine Innenbaugruppe (112) umfasst; der Griff (111) einen Montagehohlraum (111a) und eine Aufnahmeöffnung (111b) definiert, wobei die Aufnahmeöffnung (111b) mit dem Montagehohlraum (111a) verbunden ist; die Innenbaugruppe (112) eine Abtriebswelle (1121) umfasst, wobei die Innenbaugruppe (112) in dem Montagehohlraum (111a) montiert ist und die Abtriebswelle (1121) sich aus dem Montagehohlraum (111a) heraus erstreckt;
wobei das Pflegeelement (120) mit einem Ende der Abtriebswelle (1121) verbunden ist, das sich aus dem Montagehohlraum (111a) heraus erstreckt;
wobei die Energieempfangsvorrichtung (130) mit der Innenbaugruppe (112) oder dem Griff (111) verbunden ist; die Energieempfangsvorrichtung (130) entsprechend der Aufnahmeöffnung (111b) angeordnet ist, wobei die Energieempfangsvorrichtung (130) dazu konfiguriert ist, Körpermerkmalsinformationen zu erfassen; die Energieempfangsvorrichtung (130) einen vorbestimmten Erfassungsbereich aufweist; die Energieempfangsvorrichtung (130) eine Kamera umfasst, wobei die Kamera eine optische Achse (111h) definiert, und die Abtriebswelle (1121) eine Achse definiert, wobei
die optische Achse (111h) der Kamera relativ zu der Achse (112a) der Abtriebswelle (1121) in einer Richtung von dem Griff (111) zu dem Pflegeelement (120) um einen Winkel abgelenkt ist, sodass das Pflegeelement (120) außerhalb des vorbestimmten Erfassungsbereichs angeordnet ist.

2. Mundpflegevorrichtung (100) nach Anspruch 1, wobei die Mundpflegevorrichtung (100) ferner eine Energieübertragungsvorrichtung (150) umfasst; die Energieübertragungsvorrichtung (150) mit der Innenbaugruppe (112) oder dem Griff (111) verbunden ist, und die Energieübertragungsvorrichtung (150) entsprechend der Aufnahmeöffnung (111b) angeordnet ist;
wobei die Energieübertragungsvorrichtung (150) dazu konfiguriert ist, Energie an einen menschlichen Körper zu übertragen, und die Energieempfangsvorrichtung (130) dazu konfiguriert ist, zumindest reflektierte Energie der von der Energieübertragungsvorrichtung (150) übertragenen Energie zu empfangen; wobei die Energieübertragungsvorrichtung (150) einen vorbestimmten Übertragungsbereich aufweist, und das Pflegeelement (120) außerhalb des vorbestimmten Übertragungsbereichs angeordnet ist.

3. Mundpflegevorrichtung (100) nach Anspruch 2, wobei der vorbestimmte Übertragungsbereich innerhalb des vorbestimmten Erfassungsbereichs liegt.

4. Mundpflegevorrichtung (100) nach Anspruch 1, wobei ein eingeschlossener Winkel zwischen der optischen Achse der Kamera und der Achse der Abtriebswelle nicht weniger als F Grad und nicht größer als F+10 Grad beträgt, und F die Hälfte eines Bildfeldwinkels der Kamera ist.

5. Mundpflegevorrichtung (100) nach Anspruch 1, wobei die optische Achse der Kamera relativ zu dem Pflegeelement (120) nach außen geneigt ist, so dass das Pflegeelement (120) außerhalb des vorbestimmten Erfassungsbereichs angeordnet ist.

6. Mundpflegevorrichtung (100) nach Anspruch 1, wobei die Mundpflegevorrichtung (100) ferner einen Mehrachsensensor umfasst, und der Mehrachsensensor dazu konfiguriert ist, Haltungsinformationen der Mundpflegevorrichtung zu erfassen.

7. Mundpflegevorrichtung (100) nach Anspruch 1, wobei in einer Umfangsabmessung des Griffs eine Fläche, an der eine Taste angeordnet ist, oder eine dem Pflegeelement zugewandte Fläche des Pflegeelements (120) als eine Vorderseite des Griffs (111) definiert ist, und die Energieempfangsvorrichtung (130) auf einer Rückseite des Griffs (111) angeordnet ist.

8. Mundpflegevorrichtung (100) nach Anspruch 1, wobei die Innenbaugruppe (112) umfasst: eine Halterung (180); die Kamera auf der Halterung (180) angeordnet ist, und eine Lichteintrittsfläche der Kamera direkt der Aufnahmeöffnung (111b) zugewandt ist.

9. Mundpflegevorrichtung (100) nach Anspruch 1, wobei der Griff (111) ferner eine mit dem Montagehohlraum (111a) verbundene Montageöffnung (111e) umfasst; die Energieempfangsvorrichtung (130) auf der Innenbaugruppe (112) angeordnet ist, und die Energieempfangsvorrichtung (130) und die Innenbaugruppe (112) von der Montageöffnung (111e) aus in dem Montagehohlraum (111a) montiert sind.

10. Mundpflegevorrichtung (100) nach Anspruch 1, wobei der Griff (111) einen Übergangsabschnitt (1111) und einen Halteabschnitt (1112) umfasst; der Übergangsabschnitt (1111) ein erstes Verbindungsende (1113) und ein zweites Verbindungsende (1114) umfasst, wobei das zweite Verbindungsende (1114) des Übergangsabschnitts (1111) näher an dem Pflegeelement (120) liegt als das erste Verbindungsende (1113) des Übergangsabschnitts (1111); der Halteabschnitt (1112) mit dem ersten Verbindungsende (1113) des Übergangsabschnitts (1111) verbunden ist, und Flächen von Querschnitten, die senkrecht zu einer Längenrichtung der Mundpflegevorrichtung (100) stehen, des Halteabschnitts (1112) von dem ersten Verbindungsende (1113) des Übergangsabschnitts (1111) zu dem zweiten Verbindungsende (1114) des Übergangsabschnitts (1111) allmählich abnehmen;
wobei die Aufnahmeöffnung (111b) in dem Übergangsabschnitt (1111) angeordnet ist, die Aufnahmeöffnung (111b) in dem Halteabschnitt (1112) angeordnet ist, oder die Aufnahmeöffnung (111b) an einer Verbindungsstelle des Übergangsabschnitts (1111) und des Halteabschnitts (1112) angeordnet ist;
wobei die Aufnahmeöffnung (111b) von dem Pflegeelement (120) beabstandet ist.

11. Mundpflegevorrichtung (100) nach Anspruch 1, wobei die Griffbaugruppe (110) ferner eine vorstehende Struktur (113) umfasst; die vorstehende Struktur (113) von einer Außenumfangswand des Griffs (111) entlang einer Radialrichtung des Griffs (111) vorsteht, und die Aufnahmeöffnung (111b) in einer Oberfläche der vorstehenden Struktur (113) definiert ist, die dem Pflegeelement (130) zugewandt ist.

12. Mundpflegevorrichtung (100) nach Anspruch 1, wobei die Mundpflegevorrichtung (100) ein lichtdurchlässiges Stück (140) umfasst; das lichtdurchlässige Stück (140) die Aufnahmeöffnung (111b) abdeckt, um die Aufnahmeöffnung (111b) abzudichten, und das lichtdurchlässige Stück (140) geneigt ist, um Flüssigkeit oder Schaum zu leiten, um aus dem lichtdurchlässigen Stück (140) herauszufließen.

13. Mundpflegevorrichtung (100) nach Anspruch 1, wobei die Mundpflegevorrichtung (100) ein lichtdurchlässiges Stück (140) umfasst; das lichtdurchlässige Stück (140) die Aufnahmeöffnung (111b) abdeckt, um die Aufnahmeöffnung (111b) abzudichten, und eine hydrophobe Schicht (141) auf einer Außenoberfläche des lichtdurchlässigen Stücks (140) aufgebracht ist.

14. Mundpflegevorrichtung (100) nach Anspruch 12 oder Anspruch 13, wobei die Kamera direkt der Aufnahmeöffnung (111b) zugewandt ist, und ein Neigungswinkel der Kamera gleich einem Neigungswinkel des lichtdurchlässigen Stücks (140) ist.

15. Mundpflegevorrichtung (100) nach Anspruch 1, wobei die Mundpflegevorrichtung (100) ferner eine Steuerung umfasst; die Steuerung in dem Griff (111) angeordnet und mit der Energieempfangsvorrichtung (130) elektrisch verbunden ist, und die Steuerung dazu konfiguriert ist, basierend auf den durch die Energieempfangsvorrichtung (130) erfassten Körpermerkmalsinformationen eine Position des Pflegeelements (120) in einer Mundhöhle zu bestimmen; oder
wobei die Mundpflegevorrichtung (100) ferner ein Kommunikationsmodul umfasst; das Kommunikationsmodul dazu konfiguriert ist, die durch die Energieempfangsvorrichtung (130) erfassten Körpermerkmalsinformationen an ein externes Endgerät zu senden, und das externe Endgerät dazu konfiguriert ist, basierend auf den durch die Energieempfangsvorrichtung (130) erfassten Körpermerkmalsinformationen die Position des Pflegeelements (120) in der Mundhöhle zu bestimmen.

## Revendications

1. Un dispositif de soins buccaux (100), comprenant:
un ensemble manche (110);
un élément de soin (120); et
un dispositif de réception d'énergie (130);
dans lequel l'ensemble manche (110) comprend un manche (111) et un ensemble interne (112), le manche (111) définit une cavité de montage (111a) et un orifice de réception (111b), l'orifice de réception (111b) est relié à la cavité de montage (111a), l'ensemble interne (112) comprend un arbre de sortie (1121), l'ensemble interne (112) est monté dans la cavité de montage (111a) et l'arbre de sortie (1121) s'étend hors de la cavité de montage (111a);
dans lequel l'élément de soin (120) est relié à une extrémité de l'arbre de sortie (1121) s'étendant hors de la cavité de montage (111a);
dans lequel le dispositif de réception d'énergie (130) est relié à l'ensemble interne (112) ou au manche (111), le dispositif de réception d'énergie (130) est disposé de manière correspondante à l'orifice de réception (111b), le dispositif de réception d'énergie (130) est configuré pour acquérir des informations sur les caractéristiques du corps humain, le dispositif de réception d'énergie (130) présente une plage de réception prédéterminée, dans lequel le dispositif de réception d'énergie (130) comprend une caméra, la caméra définissant un axe optique (111h), et l'arbre de sortie (1121) définit un axe, dans lequel
l'axe optique (111h) de la caméra est dévié selon un angle par rapport à l'axe (112a) de l'arbre de sortie (1121) dans une direction allant du manche (111) vers l'élément de soin (120) de telle sorte que l'élément de soin (120) est disposé à l'extérieur de la plage de réception prédéterminée.

2. Le dispositif de soins buccaux (100) selon la revendication 1, dans lequel le dispositif de soins buccaux (100) comprend en outre un dispositif de transmission d'énergie (150), le dispositif de transmission d'énergie (150) est relié à l'ensemble interne (112) ou au manche (111), et le dispositif de transmission d'énergie (150) est disposé de manière correspondante à l'orifice de réception (111b);
dans lequel le dispositif de transmission d'énergie (150) est configuré pour transmettre de l'énergie à un corps humain, le dispositif de réception d'énergie (130) est configuré pour au moins recevoir l'énergie réfléchie de l'énergie transmise par le dispositif de transmission d'énergie (150), le dispositif de transmission d'énergie (150) présente une plage de transmission prédéterminée, et l'élément de soin (120) est disposé à l'extérieur de la plage de transmission prédéterminée.

3. Le dispositif de soins buccaux (100) selon la revendication 2, dans lequel la plage de transmission prédéterminée se situe à l'intérieur de la plage de réception prédéterminée.

4. Le dispositif de soins buccaux (100) selon la revendication 1, dans lequel un angle inclus entre l'axe optique de la caméra et l'axe de l'arbre de sortie n'est pas inférieur à F degrés et n'est pas supérieur à F+10 degrés, et F correspond à la moitié d'un angle de champ de la caméra.

5. Le dispositif de soins buccaux (100) selon la revendication 1, dans lequel l'axe optique de la caméra est incliné vers l'extérieur par rapport à l'élément de soin (120), de sorte que l'élément de soin (120) est disposé à l'extérieur de la plage de réception prédéterminée.

6. Le dispositif de soins buccaux (100) selon la revendication 1, dans lequel le dispositif de soins buccaux (100) comprend en outre un capteur multi-axes, le capteur multi-axes étant configuré pour obtenir des informations d'attitude du dispositif de soins buccaux.

7. Le dispositif de soins buccaux (100) selon la revendication 1, dans lequel, dans une dimension circonférentielle du manche, une surface où un bouton est disposé ou une surface orientée vers le soin de l'élément de soin (120) est définie comme un côté avant du manche (111), et le dispositif de réception d'énergie (130) est disposé sur un côté arrière du manche (111).

8. Le dispositif de soins buccaux (100) selon la revendication 1, dans lequel l'ensemble interne (112) comprend: un support (180), la caméra est disposée sur le support (180), et une surface d'incidence de lumière de la caméra fait directement face à l'orifice de réception (111b).

9. Le dispositif de soins buccaux (100) selon la revendication 1, dans lequel le manche (111) comprend en outre une ouverture de montage (111e) reliée à la cavité de montage (111a), le dispositif de réception d'énergie (130) est disposé sur l'ensemble interne (112), et le dispositif de réception d'énergie (130) et l'ensemble interne (112) sont montés dans la cavité de montage (111a) par l'ouverture de montage (111e).

10. Le dispositif de soins buccaux (100) selon la revendication 1, dans lequel le manche (111) comprend une section de transition (1111) et une section de préhension (1112), la section de transition (1111) comprend une première extrémité de connexion (1113) et une seconde extrémité de connexion (1114), la seconde extrémité de connexion (1114) de la section de transition (1111) est plus proche de l'élément de soin (120) que la première extrémité de connexion (1113) de la section de transition (1111), la section de préhension (1112) est reliée à la première extrémité de connexion (1113) de la section de transition (1111), et les aires de sections transversales, perpendiculaires à une direction de longueur du dispositif de soins buccaux (100), de la section de préhension (1112) diminuent progressivement de la première extrémité de connexion (1113) de la section de transition (1111) à la seconde extrémité de connexion (1114) de la section de transition (1111);
dans lequel l'orifice de réception (111b) est disposé dans la section de transition (1111), l'orifice de réception (111b) est disposé dans la section de préhension (1112), ou l'orifice de réception (111b) est disposé à une jonction de la section de transition (1111) et de la section de préhension (1112);
dans lequel l'orifice de réception (111b) est espacé de l'élément de soin (120).

11. Le dispositif de soins buccaux (100) selon la revendication 1, dans lequel l'ensemble manche (110) comprend en outre une structure en saillie (113), la structure en saillie (113) fait saillie depuis une paroi périphérique extérieure du manche (111) le long d'une direction radiale du manche (111), et l'orifice de réception (111b) est défini dans une surface de la structure en saillie (113) faisant face à l'élément de soin (130).

12. Le dispositif de soins buccaux (100) selon la revendication 1, dans lequel le dispositif de soins buccaux (100) comprend une pièce perméable à la lumière (140), la pièce perméable à la lumière (140) recouvre l'orifice de réception (111b) pour sceller l'orifice de réception (111b), et la pièce perméable à la lumière (140) est inclinée pour guider le liquide ou la mousse à s'écouler hors de la pièce perméable à la lumière (140).

13. Le dispositif de soins buccaux (100) selon la revendication 1, dans lequel le dispositif de soins buccaux (100) comprend une pièce perméable à la lumière (140), la pièce perméable à la lumière (140) recouvre l'orifice de réception (111b) pour sceller l'orifice de réception (111b), et un film hydrophobe (141) est appliqué sur une surface extérieure de la pièce perméable à la lumière (140).

14. Le dispositif de soins buccaux (100) selon la revendication 12 ou la revendication 13, dans lequel la caméra fait directement face à l'orifice de réception (111b), et un angle d'inclinaison de la caméra est le même qu'un angle d'inclinaison de la pièce perméable à la lumière (140).

15. Le dispositif de soins buccaux (100) selon la revendication 1, dans lequel le dispositif de soins buccaux (140) comprend en outre un contrôleur, le contrôleur est disposé dans le manche (111) et est connecté électriquement au dispositif de réception d'énergie (130), et le contrôleur est configuré pour déterminer une position de l'élément de soin (120) dans une cavité buccale sur la base des informations de caractéristiques du corps humain acquises par le dispositif de réception d'énergie (130); ou
dans lequel le dispositif de soins buccaux (100) comprend en outre un module de communication, le module de communication est configuré pour envoyer les informations de caractéristiques du corps humain acquises par le dispositif de réception d'énergie (130) à un terminal externe, et le terminal externe est configuré pour déterminer la position de l'élément de soin (120) dans la cavité buccale sur la base des informations de caractéristiques du corps humain acquises par le dispositif de réception d'énergie (130).
